# EUROPEAN PATENT SPECIFICATION

(11) **EP 0 832 134 B1**
(45) Date of publication and mention of the grant of the patent: **26.07.2000**
(21) Application number: 96918167.6
(22) Date of filing: 05.06.1996
(51) Int. Cl.: C07K 16/46, A61K 35/14, A61K 39/395, C07K 16/28, C07K 14/735

(54) **ANTI-ALLERGY BISPECIFIC MOLECULES**
BISPEZIFISCHE MOLEKÜLE GEGEN ALLERGIE
MOLECULES BISPECIFIQUES ANTIALLERGIQUES

(30) Priority: 07.06.1995 US 479902
(43) Date of publication of application: 01.04.1998
(73) Proprietor: MEDAREX, INC., Annandale, NJ 08801-0992 (US)
(72) Inventor: GUYRE, Paul, M., Hanover, NH 03755 (US); FANGER, Michael, Lebanon, NH 03766 (US)
(74) Representative: Price, Vincent Andrew
(86) International application number: US9609071
(87) International publication number: WO9640788

(56) References cited:
- EP-A- 0 255 249
- EP-A- 0 648 499
- WO-A-91/05805
- CELL, vol. 57, 5 May 1989, CAMBRIDGE, MA, USA, pages 351-354, XP002014532 J-P. KINET: "Antibody-cell interactions"
- CRITICAL REVIEWS IN IMMUNOLOGY, vol. 12, no. 3-4, 1992, BOCA RATON, FL, USA, pages 101-124, XP002014533 M. FANGER ET AL.: "Bispecific antibodies."

### SEQUENCE LISTING

(1) GENERAL INFORMATION:
   (i) APPLICANTS: Paul M. Guyre and Michael Fanger
   (ii) TITLE OF INVENTION: Anti-Allergy Bispecific Molecules
   (iii) NUMBER OF SEQUENCES: 3
   (iv) CORRESPONDENCE ADDRESS:
      (A) ADDRESSEE: LAHIVE & COCKFIELD
      (B) STREET: 60 State Street, Suite 510
      (C) CITY: Boston
      (D) STATE: Massachusetts
      (E) COUNTRY: USA
      (F) ZIP: 02109-1875
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER: US 08/479,902
      (B) FILING DATE: 07-JUNE-1995
   (viii) ATTORNEY/AGENT INFORMATION:
      (A) NAME: Arnold, Beth E.
      (B) REGISTRATION NUMBER: 35,430
      (C) REFERENCE/DOCKET NUMBER: MXI-044
   (ix) TELECOMMUNICATION INFORMATION:
      (A) TELEPHONE: (617)227-7400
      (B) TELEFAX: (617)227-5941
(2) INFORMATION FOR SEQ ID NO:1:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..24
   (xi) SEQUENCE DESCRIPTION: SEQ ID NO:1:
   ACT CAC ACA TGC CCA CCG TGC CCA                                          24
   Thr His Thr Cys Pro Pro Cys Pro
     1               5
(2) INFORMATION FOR SEQ ID NO:2:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..19
   (xi) SEQUENCE DESCRIPTION: SEQ ID NO:2:
   ACT CAC ACA TGC CCA CCG T GAGGATCC                                       27
   Thr His Thr Cys Pro Pro
     1               5
(2) INFORMATION FOR SEQ ID NO:3:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: CDS
      (B) LOCATION: 1..34
   (xi) SEQUENCE DESCRIPTION: SEQ ID NO:3:
   ACT CAC ACA TGC TCG AGC CTT CAC GGC GGC CGC T GAGGATCC                   42
   Thr His Thr Cys Ser Ser Leu His Gly Gly Arg
     1               5                  10

## Description

The binding of an allergen to an immunoglobulin E (IgE) receptor on a mast cell initiates the release of histamines and proteases from the cytoplasmic mast cell granules and the synthesis of prostaglandins and leukotrienes that mediate the inflammatory and immediate hypersensitivity responses characteristic of allergies and asthma. Considerable evidence indicates that this response occurs when two or more high-affinity IgE receptors (Fc εR) are crosslinked via IgE molecules that in turn are bound to an allergen molecule (Metzger, H. et al., (1988) *Annu. Rev. Immunol. 4*:419-470).

Many immune system effector cells (e.g., monocytes, macrophages neutrophils, natural killer (NK) cells and dendritic cells), have surface receptors that bind the Fc portion of an immunoglobulin. When such cells encounter target cells that have been opsonized with immunoglobulin antibodies, they form conjugates, and either lyse or phagocytose the target cells, depending upon the effector cell type, the targer cell type and the specific Fc receptor type (FcR) involved.

Target cell conjugation with an effector cell and lysis has also been accomplished using bispecific molecules, comprising, for example, an anti-Fc receptor antibody and an antibody directed against a target cell epitope. Complexes of effector cells bound via their Fc receptors to bispecific molecules can specifically bind and lyse target cells which have not been opsonized, but which express the appropriate target antigen.

The binding of a bispecific molecule to an effector cell via an Fc receptor can be susceptible to inhibition by physiological concentrations of immunoglobulin (since immunoglobulins also blind to Fc receptors). However, monoclonal antibodies, which bind to a site on an Fc receptor that is distinct from the binding site for endogenous immunoglobulin, have been produced (see, for example, U.S. Patent 4,954,617; Anderson et al., *J. Biol. Chem.* 261:12856 (1986); and Shen et al., *J. Immunol*. 137:3378-3382(1986)).

WO 91/05805 and EP-A-0 255 249 relate to (i) bifunctional antibodies or heteroantibodies having an anti-FcαR and FcγR portion respectively, and a second specificity for a "target epitope" present on a target cell; (ii) a target specific effector cell expressing Fc receptor and a bifunctional or heteroantibody bound to the Fc receptor of the effector cell, and (iii) diagnostic and therapeutic methods of using these compositions.

EP-A-0 648 499 relates to a peptide consisting of a portion of a high affinity immunoglobulin E receptor a-chain (FcεRIα) capable of binding to human IgE *in vitro.*

### Summary of the Invention

The invention is defined by the claims appended hereto.

In one aspect, the invention relates to bispecific molecules, which comprise an anti-immunoglobulin E (IgE) portion and an anti-effector cell portion. In a preferred embodiment, the anti-effector cell portion specifically binds an Fc receptor (FcR) on an effector cell. Most preferably the anti-Fc receptor portion of the bispecific molecule binds an FcR at a site, which is distinct from the binding site for the Fc portion of an immunoglobulin. In another preferred embodiment, the anti-IgE portion is an Fcε receptor (FcεR) or an IgE binding fragment thereof (e.g. an IgE binding portion of an FcεR α- chain). In another aspect, the invention relates to IgE specific effector cells comprising the bispecific molecules complexed to an appropriate effector cell.

In a further aspect, the invention relates to therapeutic and prophylactic uses of the bispecific molecules and IgE specific effector cells based on their ability to bind circulating IgE molecules or allergen bound IgE *in vivo.* For example, the instant claimed bispecific molecules and IgE specific effector cells can prevent IgE binding to mast cells and basophils, thereby preventing an allergic response. In addition, bispecific molecule mediated binding of effector cells to antigen bound IgE can result in effector cell presentation of the antigen, thereby resulting in a vaccine-like effect, by directing the immune system away from production of allergen-specific IgE antibodies towards production of allergen-specific IgG antibodies leading to a permanent "cure" of the allergic response.

The biological activity of the bifunctional molecules and IgE specific effector cells mimic normal physiology. Therefore the compounds should prove to be safe and effective therapeutic agents. The above discussed and many other features and advantages of the present invention will become better understood by reference to the following Detailed Description.

### Brief Description of the Figure

The Figure is a diagram showing the nucleotide and amino acid residue sequences of a portion of the hinge region of a humanized Fcγ RI antibody, H22. [A] that was altered to produce a truncated single-sulfhydryl version [B] and then altered further to engineer two unique cloning sites [C]. Underlined nucleotides indicate changes from the previous sequence. Overlined nucleotides are the recognition sequences for the indicated restriction sites.

### Detailed Description of the Invention

As used herein, the following terms and phrases shall have the meanings set forth below:

A "bispecific molecule" shall refer to a molecule, which minimally comprises an anti-Fc receptor portion; and an anti-IgE portion. The term bispecific molecule also encompasses molecules which can recognize additional molecules (i.e. are multispecific).

An "anti-effector cell portion" refers to an antibody, a functional antibody fragment (e.g. Fab fragment) or a ligand that recognizes and binds to an effector cell. In a preferred embodiment, the anti-effector cell portion binds to an Fc receptor on an effector cell. Preferred antibodies for use in the subject invention bind the Fc receptor on an effector cell at a site which is not bound by the FcR of an endogenous immunoglobulin. Examples of epitopes to which the anti-effector cell can bind include human FcγR (i.e. FcγRI (CD64), Fcγ RII or FcγRIII), FcαR (CD89). CD33 and P155. Preferred humanized anti-FcγR monodonal antibodies are described in PCT application WO 94/10332 and U.S. Patent No. 4,954.617.

An "anti-IgE portion" refers to a ligand that recognizes and binds to IgE alone or IgE bound to an allergen. A preferred anti-IgE portion is an IgE receptor (i.e.FcεR) or an IgE binding fragment thereof (e.g. the FcεR α- chain) or an anti-IgE antibody which may or may not block binding of IgE to an IgE receptor. The nucleotide sequence of the α- chain of FcεR are disclosed, e.g. in Shimizu, A. et al., (1988) *Proc. Natl. Acad. Sci. USA 85*:1907-1911; Kinet, J.-P. et al., (1987) *Biochemistry 26*:4605-4610; International Patent Application WO 89/05352; U.S. Department of Health and Human Services Patent Application No. 07/547.892; and U.S. National Institute of Patent Application No. 07/626,704 (both U.S. patent applications being available from the National Technical Information Service).

An "effector cell" refers to an immune cell. Preferred effector cells for use in the invention are antigen presenting cells. Specific effector cells express specific Fc receptors and carry out specific immune functions. For example, monocytes, macrophages, neutrophils and dendritic cells, which express FcγRI are involved in both specific killing of target cells and presenting antigens to other components of the immune system. The expression of a particular FcR on an effector cells can be regulated by humoral factors such as cytokines. For example, expression of FcγRI has been found to be up-regulated by interferon gamma (IFN-γ). This enhanced expression increases the cytotoxic activity of Fcγ RI cells against targets.

An "IgE specific effector cell" refers to an effector cell, as previously defined, linked to a bispecific molecule, as previously defined, so that the effector cell is brought into contact with IgE alone or bound to an allergen.

An "allergen" refers to a substance that can induce an allergic or asthmatic response in a susceptible subject. The list of allergens is enormous and can include pollens, insect venoms, animal dander dust, fungal spores and drugs (e.g. penicillin). Examples of natural, animal and plant allergens include proteins specific to the following genuses: *Canine* *(Canis familiaris); Dermatophagoides (e.g. Dermatophagoides farinae); Felis (Felis domesticus); Ambrosia (Ambrosia artemiisfolia; Lolium (e.g. Lolium perenne or Lolium multiflorum); Cryptomeria (Cryptomeria japonica) ; Alternaria (Alternaria alternata); Alder; Alnus (Alnus gultinosa); Betula (Betula verrucosa); Quercus (Quercus alba); Olea (Olea europa); Artemisia (Artemisia vulgaris); Plantago* (e.g. *Plantago lanceolata); Parietaria* (e.g. *Parietaria officinalis or Parietaria judaica); Blattella* (e.g. *Blattella germanica); Apis* (e.g. *Apis multiflorum); Cupressus* (e.g. *Cupressus sempervirens, Cupressus arizonica* and *Cupressus macrocarpa); Juniperus* (e.g. *Juniperus sabinoides, Juniperus virginiana, Juniperus communis* and *Juniperus ashei) Thuya* (e.g. *Thuya orientalis); Chamaecyparis* (e.g. *Chamaecyparis obtusa); Periplaneta* (e.g. *Periplaneta americana); Agropyron* (e.g. *Agropyron repens); Secale* (e.g. *Secale cereale); Triticum* (e.g. *Triticum aestivum); Dactylis (e.g. Dactylis glomerata); Festuca (e.g. Festuca elatior); Poa* (e.g. *Poa pratensis* or *Poa compressa); Avena* (e.g. *Avena sativa); Holcus* (e.g. *Holcus lanatus); Anthoxanthum (e.g. Anthoxanthum odoratum); Arrhenatherum* (e.g. *Arrhenatherum elatius); Agrostis* (e.g. *Agrostis alba); Phleum (e.g. Phleum pratense); Phalaris* (e.g. *Phalaris arundinacea); Paspalum* (e.g. *Paspalum notatum); Sorghum* (e.g. *Sorghum halepensis);* and Bromus (e.g. *Bromus inermis).*

The bispecific molecules of the present invention can be prepared by conjugating the anti-Fc R and anti-IgE portions using methods described in the following Example or that are well-known in the art. For example, a variety of coupling or cross-linking agents can be used for covalent conjugation. Examples of cross-linking agents include protein A, carbodiimide, N-succinimidyl-S-acetyl-thioacetate (SATA), N-succinimidyl-3-(2-pyridyldithio) propionate (SPDP), and sulfosuccinimidyl 4-(N-maleimidomethyl) cyclohexane-1-carboxylate (sulfo-SMCC) (see e.g., Karpovsky et al. (1984) *J*. *Exp. Med.* 160:1686; Liu, M.A. et al. (1985) *Proc. Natl. Acad. Sci. USA* 82:8648. Other methods include those described by Paulus (*Behring Inst. Mitt.* (1985) No. 78, 118-132); Brennan et al. (*Science* (1985) 229:81-83), and Gennie et al. (*J. Immunol.* (1987) 139:2367-2375). Preferred conjugating agents are SATA and sulfo-SMCC, both available from Pierce Chemical Co. (Rockford, IL.).

Based on their ability to bind circulating IgE molecules or antigen bound IgE *in vivo,* the compounds can be administered to a subject (e.g. human or animal) for any of a number of therapeutic or prophylactic uses. For example, by preventing IgE binding to mast cells and basophils, the instant claimed molecules can thereby prevent an allergic response in a subject. In addition, bispecific molecule mediated binding of effector cells to allergen bound IgE can result in effector cell presentation of the allergen, thereby inducing a vaccine like effect against the particular allergen. In addition, bispecific molecule mediated binding of effector cells to allergen bound IgE can result in effector cell presentation of the antigen, thereby resulting in a vaccine-like effect, by directing the immune system away from production of allergen-specific IgE antibodies towards production of allergen-specific IgG antibodies leading to a permanent "cure" of the allergic response.

Bispecific molecules of the present invention can be administered freely in a physiologically acceptable solution or can first be coupled to an effector cell, forming an "IgE specific effector cell", prior to being administered to a subject. Bispecific molecules or IgE specific effector cells can be administered *in vivo* as a suspension of cells in a physiologically acceptable solution. The number of cells administered can be in the order of 10⁸-10⁹, but will vary depending on the therapeutic purpose. In general, the amount will be sufficient to obtain localization of the effector cell at IgE. The term physiologically acceptable solution, as used herein, is intended to include any carrier solution which stabilizes the bispecific molecules of IgE specific effector cells for administration *in vivo* including, for example, saline and aqueous buffer solutions, solvents, antibacterial and antifungal agents, isotonic agents, and the like. Routes of administering bispecific molecules or IgE specific effector cells to a subject can vary and include intravenous, intramuscular, and intraperitoneal administration.

The instant invention is further illustrated by the following Example, which is not intended to limit the invention in any manner.

### Example 1 Generation of Functional H22-IgER Fusion Proteins

### Materials and Methods

### A. Expression vectors and cloning

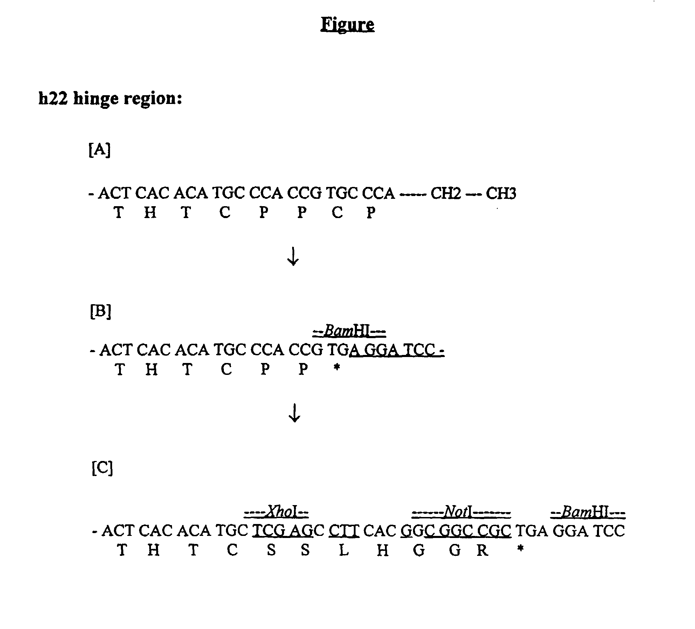

Expression vectors for the genomic clones of the heavy (pSVgpt) and light (pSVhyg) chains of H22 are as described in International Patent Application Publication Number: WO 94/10332 entitled, *Humanized Antibodies to Fc Receptors for Immunoglobulin G on Human Mononuclear Phagocytes.* For the Fab-ligand fusion construct, it is unnecessary to alter the light chain. For the heavy chain, however, the CH2 and CH3 domains must be removed and replaced with the coding sequences of the ligands. The heavy chain vector contains two *Bam*HI sites, one in the intron between VH and CH1, and the other just downstream of CH3. Using the BamHI restriction sites, DNA encoding the constant domains is replaced by a truncated version encoding only CH1 and most of the hinge. To do this, the polymerase chain reaction (PCR) is utilized to engineer the new C-terminus of the heavy chain fragment with the alterations shown in Figure 1.

The construct shown in Figure 1 [C], consists of a translation termination codon downstream of the cloning restriction sites, *Xho*I and *Not*I*,* and upstream of a *Bam*HI site which is used to clone the new PCR generated CHI fragment downstream of VH to generate the fusion protein constructs. The cloning sites, which are located downstream of most of the hinge in order to retain flexibility between the Fd and ligand domains, is used to insert DNA encoding EGF or other ligands. Also, the single Cys residue is retained from the previous construct to allow conjugation for the formation of dimeric molecules.

DNA encoding the α chain of IgER is amplified by PCR to have a *Xho*I site on the N-terminus and a *Not*I site on the C-terminus of the coding region, and then inserted in the proper reading frame into the same sites of the newly engineered H22 heavy chain truncated fragment described above. cDNA encoding the α chain of is obtained from the ATCC (#59957).

### B. Expression

The murine myeloma NSO (ECACC 85110503) is a non-Ig synthesizing line and can be used for expression of H22-IgER fusion proteins. The final expression vector, a pSVgpt construct with DNA encoding H22 Fd fused in frame to IgER is transfected by electroporation using a BioRad Gene Pulser to NS0 which had been previously transfected with the pSVhyg construct containing DNA encoding H22 light chain. These polypeptides can be expressed by an Ig promoter and Ig enhancer present in the vectors, and secreted by the mAb 22 heavy chain signal peptide located on the N-terminus of the constructs. One or two days after transfection, mycophenolic acid and xanthine can be added to the media to select for cells that took up the DNA. Individual growing colonies can be isolated and subcloned after binding activity is demonstrated by ELISA.

### C. Purification

Cells expressing the H22-IgER fusion protein can be subcloned and expanded. The fusion protein-expressing clone can be expanded and grown in spinner cultures and the supernatant clarified and concentrated. Small scale purification can be performed by affinity chromatography on an anti-human kappa chain affinity column (Sterogene; Carlsbad, CA). The purified protein can be analyzed by SDS-PAGE on a 5-15% acrylamide gradient gel under nonreducing conditions.

## Claims

1. A bispecific molecule comprising: an anti-effector cell portion and an anti-IgE portion.

2. A bispecific molecule of Claim 1, wherein the anti-IgE portion is an Fc ε receptor or an IgE binding portion of an α chain of an Fcε receptor.

3. A bispecific molecule of Claim 1 or Claim 2 wherein the anti-effector cell portion binds an Fc receptor, the anti-effector cell portion binding the Fc receptor preferably at a site that is not inhibited by endogenous immunoglobulin.

4. A bispecific molecule of Claim 3 wherein the anti-effector cell portion binds an Fcγ receptor, the Fcγ receptor being preferably selected from the group consisting of FcγRI, FCγRII, FCγRIII.

5. A bispecific molecule of Claim 3 wherein the anti-effector cell portion binds an Fcα receptor.

6. An IgE specific effector cell comprising a bispecific molecule of any one of Claims 1 to 5 wherein the anti-effector cell portion is bound to an effector cell, the effector cell being preferably an antigen presenting cell, for example selected from the group consisting of monocytes, macrophages, dendritic cells and natural killer cell.

7. The molecule of any one of Claims 1 to 5 or cell of Claim 6 for use in therapy or prophylaxis, for example in a method for preventing an allergic reaction in a subject comprising administering to the subject an effective amount of the molecule or cell.

8. The molecule of any one of Claims 1 to 5 or cell of Claim 6 for use in therapy or prophylaxis, for example in a method for directing the immune response of a subject away from production of allergen specific IgE antibodies and towards production of allergen-specific IgG antibodies, comprising administering to the subject an effective amount of the molecule or cell.

9. The bispecific molecule of Claim 1, wherein the anti-IgE portion binds to soluble IgE molecules or allergen-bound soluble IgE.

10. Use of the molecule of any one of Claims 1 to 5 or cell of Claim 6 in the manufacture of a medicament for preventing an allergic reaction in a subject or for directing the immune response of a subject away from production of allergen-specific IgE antibodies and towards production of allergen-specific IgE antibodies.

## Patentansprüche

1. Bispezifisches Molekül, das folgendes umfaßt einen Anti-Effektorzell-Anteil und einen Anti-IgE-Anteil.

2. Bispezifisches Molekül nach Anspruch 1, bei dem der Anti-IgE-Anteil ein Fcε-Rezeptor oder ein IgE-Bindungsanteil einer α-Kette eines Fcε-Rezeptors ist.

3. Bispezifisches Molekül nach Anspruch 1 oder Anspruch 2, bei dem der Anti-Effektor-Zell-Anteil einen Fc-Rezeptor bindet, wobei der Anti-Effektor-Zellanteil den Fc-Rezeptor vorzugsweise an einer Stelle bindet, die nicht durch endogenes Immunglobulin gehemmt wird.

4. Bispezifisches Molekül nach Anspruch 3, bei dem der Anti-Effektorzell-Anteil einen Fc γ-Rezeptor bindet, wobei der Fc γ-Rezeptor vorzugsweise aus der Gruppe ausgewählt ist, die aus FcγRI, FcγRII, FcγRIII besteht.

5. Bispezifisches Molekül nach Anspruch 3, bei dem der Anti-Effektorzell-Anteil einen Fcα-Rezeptor bindet.

6. IgE-spezifische Effektorzelle, die ein bispezifisches Molekül nach einem der Ansprüche 1 bis 5 umfaßt, wobei der Anti-Effektorzell-Anteil an eine Effektorzelle gebunden ist, wobei die Effektorzelle vorzugsweise eine Antigen-präsentierende Zelle ist, die beispielsweise aus der Gruppe ausgewählt ist, die aus Monozyten, Makrophagen, dendritischen Zellen und natürlichen Killerzellen besteht.

7. Molekül nach einem der Ansprüche 1 bis 5 oder Zelle nach Anspruch 6 zur Verwendung in Therapie oder Prophylaxe, beispielsweise bei einem Verfahren zur Vermeidung einer allergischen Reaktion bei einem Patienten, das die Verabreichung einer wirksamen Menge des Moleküls oder der Zelle an den Patienten umfaßt.

8. Molekül nach einem der Ansprüche 1 bis 5 oder Zelle nach Anspruch 6 zur Verwendung in Therapie oder Prophylaxe, beispielsweise bei einem Verfahren zum Weg-Wegleitung der Immunantwort eines Patienten von der Erzeugung eines allergen-spezifischen IgE-Antikörpers und hin zur Erzeugung von allergen-spezifischen IgG-Antikörpern, das die Verabreichung einer wirksamen Menge des Moleküls oder der Zelle an den Patienten umfaßt.

9. Bispezifisches Molekül nach Anspruch 1, bei dem der Anti-IgE-Anteil an lösliche IgE-Moleküle oder allergen-gebundenes lösliches IgE bindet.

10. Verwendung des Moleküls nach einem der Ansprüche 1 bis 5 oder der Zelle nach Anspruch 6 bei der Herstellung eines Medikamentes zur Vermeidung einer allergischen Reaktion bei einem Patienten oder zur Wegleitung der Immunantwort eines Patienten von der Erzeugung von allergen-spezifischen IgE-Antikörpern hin zur Erzeugung von allergen-spezifischen IgE-Antikörpern.

## Revendications

1. Une molécule bispécifique comprenant : une partie de cellule anti-effectrice et une partie anti-IgE.

2. Une molécule bispécifique selon la revendication 1, dans laquelle la partie anti-IgE est un récepteur Fcε ou une partie fixant un IgE d'une chaîne α d'un récepteur Fcε.

3. Une molécule bispécifique selon la revendication 1 ou 2 dans laquelle la partie de la cellule anti-effectrice fixe un récepteur Fc, la partie de la cellule anti-effectrice liant le récepteur Fc de préférence à un site qui n'est pas inhibé par une immunoglobuline endogène.

4. Une molécule bispécifique selon la revendication 3 dans laquelle la partie de la cellule anti-effectrice fixe un récepteur Fcγ, le récepteur Fcγ étant de préférence choisi parmi le groupe consistant en FcγRI, FcγRII, FcγRIII.

5. Une molécule bispécifique selon la revendication 3 dans laquelle la partie de la cellule anti-effectrice fixe un récepteur Fcα.

6. Une cellule effectrice spécifique d'un IgE comprenant une molécule bispécifique selon l'une quelconque des revendications 1 à 5 dans laquelle la partie de la cellule anti-effectrice est liée à une cellule effectrice, la cellule effectrice étant de préférence une cellule présentant un antigène, par exemple choisi parmi le groupe comprenant les monocytes, les macrophages, les cellules dendritiques et les cellules tueuses naturelles.

7. La molécule selon l'une quelconque des revendications 1 à 5 ou la cellule selon la revendication 6 pour une utilisation en thérapie ou prophylaxie, par exemple dans une méthode pour éviter une réaction allergique chez un sujet comprenant l'administration au sujet d'une quantité efficace de la molécule ou cellule.

8. La molécule selon l'une quelconque des revendications 1 à 5 ou la cellule selon la revendication 6 pour une utilisation en thérapie ou prophylaxie, par exemple dans une méthode pour empêcher la réponse immunitaire d'un sujet envers la production des anticorps IgE spécifiques aux allergènes et favoriser la production des anticorps IgG spécifiques aux allergènes, comprenant l'administration au sujet d'une quantité efficace de la molécule ou cellule.

9. La molécule bispécifique selon la revendication 1, dans laquelle la partie anti-IgE fixe des molécules IgE solubles ou des IgE solubles liés à des allergènes.

10. Utilisation de la molécule selon l'une quelconque des revendications 1 à 5 ou de la cellule selon la revendication 6 dans la fabrication d'un médicament pour éviter une réaction allergique chez un sujet ou pour empêcher la réponse immunitaire d'un sujet envers la production des anticorps IgE spécifiques aux allergènes et favoriser la production d'anticorps IgE spécifiques aux allergènes.
